(19) Europäisches Patentamt
European Patent Office
Office européen des brevets

(11) EP 2 328 545 B1

(12) EUROPÄISCHE PATENTSCHRIFT

(45) Veröffentlichungstag und Bekanntmachung des Hinweises auf die Patenterteilung:
**05.08.2015 Patentblatt 2015/32**

(21) Anmeldenummer: **09778036.5**

(22) Anmeldetag: **21.08.2009**

(51) Int Cl.:
***A61K 8/60*** *(2006.01)* ***A61Q 19/00*** *(2006.01)*
***A61P 17/00*** *(2006.01)*

(86) Internationale Anmeldenummer:
**PCT/EP2009/006083**

(87) Internationale Veröffentlichungsnummer:
**WO 2010/020424 (25.02.2010 Gazette 2010/08)**

(54) **VERWENDUNG VON GLUCOSYLGLYCEROL**

USE OF GLUCOSYLGLYCEROL

UTILISATION DE GLUCOSYLGLYCEROL

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **22.08.2008 DE 102008039231**

(43) Veröffentlichungstag der Anmeldung:
**08.06.2011 Patentblatt 2011/23**

(73) Patentinhaber: **Bitop AG**
**58453 Witten (DE)**

(72) Erfinder:
- **KLEIN, Julia**
**44869 Bochum (DE)**
- **STUMM, Gerhard**
**22143 Hamburg (DE)**

(74) Vertreter: **Schöneborn, Holger**
**Schneiders & Behrendt**
**Rechts- und Patentanwälte**
**Huestraße 23**
**44787 Bochum (DE)**

(56) Entgegenhaltungen:
**WO-A1-2009/056292**
**DE-A1-102005 023 636**
**DE-A1-102005 023 639**
**DE-A1-102005 023 640**
**JP-A- 2004 331 578**
**JP-A- 2004 331 582**

EP 2 328 545 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft die Verwendung von Glucosylglycerol im Rahmen von kosmetischen oder dermatologischen Zubereitungen.

In der DE 195 40 749 A1 wird die Verwendung von Glycosylglyceriden in kosmetischen und dermatologischen Zubereitungen beschrieben. Derartige Substanzen lassen sich als sog. Moisturizer, d. h. als feuchtigkeitsspendende Substanzen, einsetzen. Besonders bevorzugt ist dabei die Verwendung von 2-O-β-D-Glucosylglycerol.

Glucosylglycerol, genauer 2-O-α-D-Glucosylglycerol, ist als Naturstoff bekannt und wird beispielsweise von Cyanobakterien synthetisiert, denen es als osmoprotektive Substanz dient. Auf diese Weise gestattet es den Cyanobakterien das Wachstum in salzhaltigen Medien mit einer Konzentration von bis zu 1,5 M NaCl. Das Molekül wird in hohen Konzentrationen im Cytoplasma akkumuliert, um auf diese Weise den aufgrund der hohen Salzkonzentration in der Umgebung vorliegenden osmotischen Druck zu verringern und die Zelle vor Wasserverlust zu schützen. Ein Beispiel ist das Cyanobakterium *Synechocystis* sp. PCC 6803.

Darüber hinaus wird das Molekül auch von Pflanzen der Gattung Myrothamnus synthetisiert. Bei diesen Pflanzen handelt es sich um wechselfeuchte Pflanzen. *Myrothamnus flabeilifolia* ist ein kleiner Strauch aus dem südlichen Afrika, der auf Felsplatten wächst und bis zu 60 cm hoch wird. Die im südlichen Afrika auftretenden mehrere Monate andauernden Dürreperioden übersteht die Pflanze unbeschadet in ausgetrocknetem Zustand. Sobald es jedoch regnet, wird die Pflanze innerhalb weniger Stunden wieder grün, woraus sich auch der Beiname "Auferstehungspflanze" ergibt. Die Struktur von 2-O-α-D-Glucosylglycerol ist wie folgt:

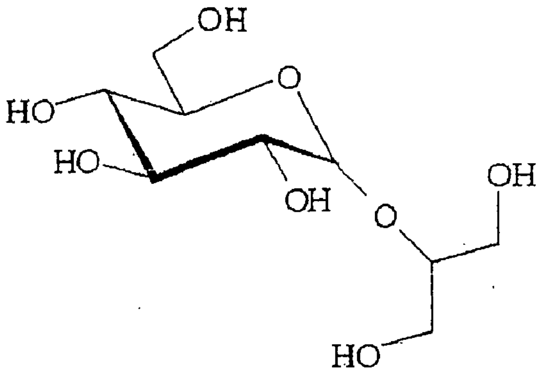

Aus der JP 2004-331578 A ist die Verwendung von Glucosylglycerolen in Hautpflegemitteln zur Bekämpfung der Hautalterung bekannt.

Überraschend hat sich nunmehr herausgestellt, dass Glucosylglycerole in der Lage sind, die Expression von Zellschutzenzymen zu steigern, was in Versuchen mit menschlichen Hautzellen nachgewiesen werden konnte. Die Erfindung betrifft daher die Verwendung von Glucosylglycerol zur Herstellung eines Mittels zum Schutz der menschlichen Haut und/oder Schleimhaut vor reaktiven Sauerstoffverbindungen, freien Radikalen, Chemikalien, Toxinen, Allergenen, denaturierenden Substanzen, an die DNA bindenden Substanzen, proteinschädigenden Substanzen, proteininaktivierenden Substanzen oder Schwebstäuben durch Steigerung der Expression von Zellschutzenzymen.

Die Wirkung von Glucosylglycerol konnte anhand von Versuchen mit Keratinozyten und Fibroblasten nachgewiesen werden. Bei den Versuchen wurden entsprechende Zellkulturen mit einer Glucosylglycerol-Lösung behandelt und die transkribierte mRNA quantifiziert. Hierzu wurde die mRNA zunächst extrahiert, um mit Hilfe einer Reverse Transkriptase ein $^{33}P$-gelabeltes Target zu erzeugen. Anschließend wurden diese Targets auf einen cDNA-Chip aufgebracht und die Radioaktivität mittels Phosphorimaging gemessen. Der cDNA-Chip enthielt ein Array der cDNAs verschiedener Proteine.

Hierbei hat sich herausgestellt, dass die Expression von Zellschutzenzymen hochreguliert wird. Unter Zellschutzenzymen werden insbesondere solche Enzyme verstanden, die in der Lage sind, reaktive Sauerstoffverbindungen abzubauen. Ein Beispiel hierfür ist die Superoxiddismutase, bei welcher es sich um ein Enzym handelt, das eukaryotische Zellen vor reaktiven Superoxidionen schützt. Hierbei reagiert die oxidierte Form des Enzyms mit einem Superoxidanion unter Bildung von Sauerstoff und der reduzierten Form des Enzyms. Diese Form wiederum reagiert mit einem zweiten Superoxidanion unter Bildung von Wasserstoffperoxid, wobei sich die oxidierte Form des Enzyms zurückbildet. Insgesamt ergibt sich damit folgende Gleichung: $2O_2^- + 2H^+ \rightarrow H_2O_2 + O_2$

Ein weiteres in diesem Zusammenhang wichtiges Enzym ist die Katalase. Diese disproportioniert Wasserstoffperoxid zu Sauerstoff und Wasser. Die Katalase vermindert auf diese Weise, ähnlich wie die Superoxiddismutase, den

oxidativen Stress der Hautzellen.

Auch die Glutathionperoxidase ist bei der zellulären Abwehr gegen die Folgen von oxidativem Stress von Bedeutung. Die Glutathionperoxidase katalysiert die Glutathion-abhängige Reduktion von organischen Peroxiden und Wasserstoffperoxid.

Die genannten Zellschutzenzyme, die in der Lage sind, reaktive Sauerstoffverbindungen abzubauen, insbesondere das Superoxidanion, Hydroxyradikale und Peroxide, spielen eine wichtige Rolle beim Schutz vor oxidativem Stress. Die Haut/Schleimhaut ist als Grenzschicht und Oberfläche des menschlichen Körpers einer Vielzahl externer Stressfaktoren ausgesetzt. Es handelt sich bei der menschlichen Haut um ein Organ, das mit verschiedenen spezialisierten Zelltypen - den Keratinozyten, Melanozyten, Langerhanszellen, Merkel-Zellen und anderen - den Körper vor äußeren Einflüssen schützt. Hierbei ist zwischen physikalischen, chemischen und biologischen Einflüssen auf die menschliche Haut zu unterscheiden. Zu den physikalischen Einflüssen sind thermische und mechanische Einflüsse sowie die Einwirkung von Strahlung, z. B. UV-, VIS- und IR-Strahlung, zu zählen. Unter den chemischen Einflüssen sind insbesondere die Einwirkung von Chemikalien, Toxinen, freien Radikalen, Allergenen, denaturierenden Substanzen, an die DNA bindenden Substanzen und proteinschädigenden oder proteininaktivierenden Substanzen zu nennen. Auch Schwebstäube können schädigende Wirkungen entfalten. Die äußeren biologischen Einflüsse umfassen die Einwirkung fremder Organismen und deren Stoffwechselprodukte. Auch Hitze und Kälte können die menschliche Haut angreifen. Die erfindungsgemäße Verwendung von Glucosylglycerol kann die Haut vor derartigen Einflüssen schützen.

Die Stimulierung und Aktivierung von Zellschutzenzymen konnte insbesondere anhand der Superoxiddismutasen SOD-1 und SOD-2 gezeigt werden. Die Expression der SOD-1 konnte im Falle von Keratinozyten innerhalb von 24 Stunden bei Anwendung einer 0,5 %igen Glucosylglycerol-Lösung um das 4,7-, innerhalb von 96 Stunden um das 19,6-fache gesteigert werden. Im Falle der SOD-2 ergab sich bei Tests mit Fibroblasten und Anwendung einer 1 %igen Glucosylglycerol-Lösung innerhalb von 24 Stunden eine Steigerung um das 25,4-, innerhalb von 96 Stunden um 34,4-fache.

Neben reaktive Sauerstoffverbindungen abbauenden Enzymen können auch andere Zellschutzenzyme hochreguliert werden, beispielsweise DNAreparierende Enzyme wie Ligasen. Eine weitere Klasse stellen die Chaperone dar, die Proteinen helfen, sich korrekt zu falten.

Bevorzugt handelt es sich bei dem Glucosylglycerol um das natürlich vorkommende 2-O-α-D-Glucosylglycerol, wie es beispielsweise von Cyanobakterien der Gattung *Synechocystis* akkumuliert wird. Entsprechende Wirkungen können jedoch auch bei β-glycosidischer Verknüpfung der Glucose mit dem Glycerin-Molekül oder bei Verknüpfung der Glucose mit dem Glycerin in 1-Position erwartet werden. Im einzelnen sind somit die folgenden Glucosylglycerole denkbar, wobei hier jeweils nur die Moleküle in der D-Konfiguration dargestellt sind:

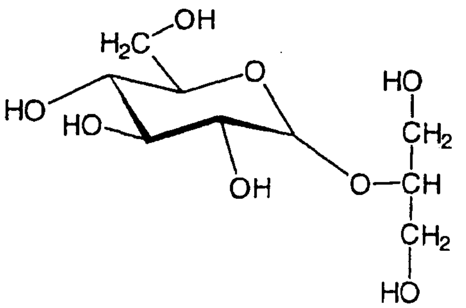

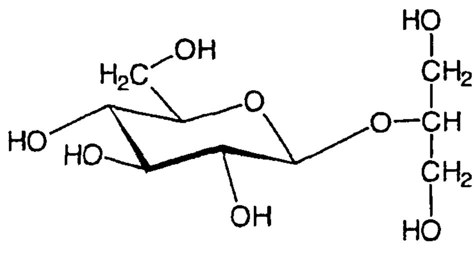

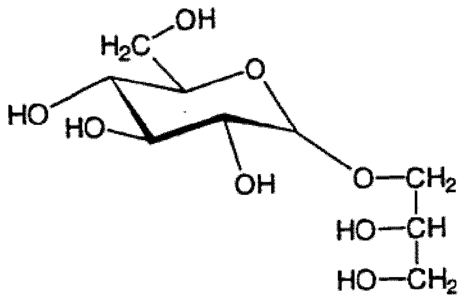

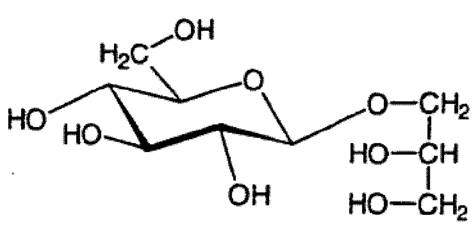

Das Glucosylglycerol kann für die beschriebenen Zwecke in kosmetischen, dermatologischen und pharmazeutischen Zubereitungen eingesetzt werden. Die Konzentration kann z. B. in einem Bereich von 0,001 Gew.-% bis 10 Gew.-%, insbesondere 0,01 Gew.-% bis 6 Gew.-%, bezogen auf das Gesamtgewicht der Zubereitung, liegen.

Das Glucosylglycerol liegt insbesondere in wässriger Lösung vor. Denkbar sind jedoch grundsätzlich auch

Emulsionen und Mikroemulsionen vom Typ Wasser-in-Öl (W/O) oder vom Typ Öl-in-Wasser (O/W).

Möglich ist der Einsatz von üblichen kosmetischen Hilfsstoffen, z. B. Trägermitteln, Konservierungsmitteln, Bakteriziden, Parfümen, Lösungsvermittlern, Vitaminen, Stabilisatoren, Substanzen zum Verhindern des Schäumens, Verdickungsmitteln, Farbstoffen, oberflächenaktiven Substanzen, Emulgatoren, feuchthaltenden Substanzen u.ä.

Die Glucosylglycerol enthaltenden kosmetischen oder dermatologischen Formulierungen sind für die äußerliche Anwendung vorgesehen. Als Anwendungsform seien z. B. genannt: Lösungen, Suspensionen, Emulsionen, Pasten, Salben, Gele, Cremes, Lotionen, Puder, Seifen, tensidhaltige Reinigungspräparate, Öle, Sprays und Lippenstifte.

Salben, Pasten, Cremes und Gele können die üblichen Trägerstoffe enthalten, z. B. tierische und pflanzliche Fette, Wachse, Paraffine, Stärke, Traganth, Zellulosederivate, Polyethylenglykole, Silikone, Bentonite, Kieselsäure, Talkum und Zinkoxid oder Gemische dieser Stoffe.

Puder und Sprays können zusätzlich zu den üblichen Trägerstoffen die üblichen Treibmittel, z. B. Propan/Butan oder Dimethylether, enthalten.

Lösungen und Emulsionen können übliche Trägerstoffe, wie Lösungsmittel, Lösungsvermittler und Emulgatoren oder Öle enthalten.

Suspensionen enthalten typischerweise zusätzliche Trägerstoffe wie Wasser oder Ethanol.

Die Herstellung des Glucosylglycerols kann gemäß einem Verfahren erfolgen, wie es in der WO 2008/034158 A2 beschrieben ist. Hier lässt man eine Saccharose-Phosphorylase auf eine Mischung einwirken, die einen Glucosyl-Donor und Glycerin als Glucosyl-Akzeptor aufweist. Bevorzugt handelt es sich bei dem Glucosyl-Donor um Saccharose.

Die Steigerung der Expression von Zellschutzenzymen konnte wie folgt gezeigt werden:

Die Untersuchungen wurden anhand von Epidermiskeratinozyten (NHEK, Normal Human Epidermal Keratinocytes) und dermalen Fibroblasten (NHDF, Normal Human Dermal Fibroblasts) durchgeführt. Diese wurden im Falle der Keratinocyten mit einer 0,5 %igen (w/w), im Falle der Fibroblasten mit einer 1 %igen (w/w) wässrigen Glucosylglycerin-Lösung behandelt.

<u>Kulturbedingungen:</u>

37°C, 5 % $CO_2$

<u>Kulturmedium:</u>

Keratinocyten: Keratinocyte-SFM (Invitrogen 17005-034) versetzt mit Epidermal Groth Factor (EGF) 0,25 ng/ml, Hypophysenextrakt (PE) 25 μg/ml (Invitrogen 3700015), Gentamycin 25 μm/ml (Sigma G1397)

Fibroblasten: DMEM (Invitrogen 21969035), versetzt mit L-Glutamin 2 mM (Invitrogen 25030024), Penicillin 50 Ul/ml/Streptomycin 50 μg/ml (Invitrogen 15070063), Fetales Kalbsserum 10 % (FCS, Invitrogen 10270098)

Die Kultivierung erfolgte für 24 und 96 Stunden. Am Ende der Inkubationszeit wurden die Zellen mit PBS-Lösung (Invitrogen 14190094) gewaschen.

Die Extraktion der mRNA jeder Kultur erfolgte mit TriReagent gemäß einem Standardprotokoll. Die entsprechenden cDNAs mit $^{33}P$-gelabelten Targets wurden durch reverse Transkription der mRNA unter Verwendung von [$\alpha^{33}P$]-dATP und oligodT hergestellt.

Die gelabelten cDNA-Targets wurden mit spezifischen cDNA-Sonden hybridisiert, die kovalent auf Minichips fixiert waren. Nach intensivem Waschen wurde die relative Menge der hybridisierten Targets mittels Phosphorimaging ermittelt. Diese Analyse erfolgte durch Messung der Radioaktivität mit Hilfe eines "Cyclone" PhosphorImager (Packard Instruments; 72 Stunden Exposition) unter Verwendung von ImageQuant TL-Software (Amersham Biosiences).

Es wurden folgende Ergebnisse erhalten:

<u>Hochregulierung der cytosolischen SOD-1 bei Keratinocyten</u>

24 h:

Kontrolle: 18,2

mit Glucosylglycerin-Lösung behandelt: 85

96 h:

Kontrolle: 9,3
mit Glucosylglycerin-Lösung behandelt: 182

Hochregulierung der SOD-2 bei Fibroblasten

24 h:

Kontrolle: 16,5
mit Glucosylglycerin-Lösung behandelt: 419

96 h:

Kontrolle: 9,1
mit Glucosylglycerin-Lösung behandelt: 313

## Patentansprüche

1. Verwendung von Glucosylglycerol zur Herstellung eines Mittels zum Schutz der menschlichen Haut und/oder Schleimhaut vor reaktiven Sauerstoffverbindungen, freien Radikalen, Chemikalien, Toxinen, Allergenen, denaturierenden Substanzen, an die DNA bindenden Substanzen, proteinschädigenden Substanzen, proteininaktivierenden Substanzen oder Schwebstäuben durch Steigerung der Expression von Zellschutzenzymen.

2. Verwendung nach Anspruch 1, **dadurch gekennzeichnet, dass** das Glucosylglycerol 2-O-α-D-Glucosylglycerol ist.

3. Verwendung nach Anspruch 1 oder 2, **dadurch gekennzeichnet, dass** das Zellschutzenzym ein reaktive Sauerstoffverbindungen abbauendes Enzym ist.

4. Verwendung nach Anspruch 3, **dadurch gekennzeichnet, dass** das Enzym eine Superoxiddismutase, eine Katalase oder eine Glutathionperoxidase ist.

5. Verwendung nach Anspruch 4, **dadurch gekennzeichnet, dass** die Superoxiddismutase die SOD-1 oder SOD-2 ist.

6. Verwendung nach Anspruch 1 oder 2, **dadurch gekennzeichnet, dass** das Zellschutzenzym ein DNA-Reparaturenzym, insbesondere eine Ligase, ist.

7. Verwendung nach Anspruch 1 oder 2, **dadurch gekennzeichnet, dass** das Zellschutzenzym ein Chaperon ist.

8. Verwendung nach einem der Ansprüche 1 bis 7, **dadurch gekennzeichnet, dass** das Glucosylglycerol in wässriger Lösung vorliegt.

9. Verwendung nach einem der Ansprüche 1 bis 8, **dadurch gekennzeichnet, dass** die Konzentration des Glucosylglycerols 0,001 Gew.-% bis 10 Gew.-%, insbesondere 0,01 Gew.-% bis 6 Gew.-% beträgt.

10. Glucosylglycerol zum Schutz der menschlichen Haut und/oder Schleimhaut vor reaktiven Sauerstoffverbindungen, freien Radikalen, Chemikalien, Toxinen, Allergenen, denaturierenden Substanzen, an die DNA bindenden Substanzen, proteinschädigenden Substanzen, proteininaktivierenden Substanzen oder Schwebstäuben durch Steigerung der Expression von Zellschutzenzymen.

11. Glucosylglycerol nach Anspruch 10, **dadurch gekennzeichnet, dass** das Glucosylglycerol 2-O-α-D-Glucosylglycerol ist.

12. Glucosylglycerol nach Anspruch 10 oder 11, **dadurch gekennzeichnet, dass** das Zellschutzenzym ein reaktive Sauerstoffverbindungen abbauendes Enzym ist.

13. Glucosylglycerol nach Anspruch 12, **dadurch gekennzeichnet, dass** das Enzym eine Superoxiddismutase, eine

Katalase oder eine Glutathionperoxidase ist.

14. Glucosylglycerol nach Anspruch 13, **dadurch gekennzeichnet, dass** die Superoxiddismutase die SOD-1 oder SOD-2 ist.

15. Glucosylglycerol nach Anspruch 10 oder 11, **dadurch gekennzeichnet, dass** das Zellschutzenzym ein DNA-Reparaturenzym, insbesondere eine Ligase, oder ein Chaperon ist.

## Claims

1. Use of glucosylglycerol for the manufacture of an agent for the protection of human skin and/or mucous membrane against reactive oxygen compounds, free radicals, chemicals, toxins, allergens, denaturing substances, substances binding to DNA, substances damaging proteins, substances deactivating proteins, or airborne particulate by increasing the expression of cell protective enzymes.

2. Use according to claim 1, **characterized in that** the glucosylglycerol is 2-O-α-D-glucosylglycerol.

3. Use according to claim 1 or 2, **characterized in that** the cell protective enzyme is an enzyme decomposing reactive oxygen compounds.

4. Use according to claim 3, **characterized in that** the enzyme is a superoxide dismutase, a catalase or a glutathione peroxidase.

5. Use according to claim 4, **characterized in that** the superoxide dismutase is SOD-1 or SOD-2.

6. Use according to claim 1 or 2, **characterized in that** the cell protective enzyme is a DNA repairing enzyme, in particular a ligase.

7. Use according to claim 1 or 2, **characterized in that** the cell protective enzyme is a chaperone.

8. Use according to any one of claims 1 to 7, **characterized in that** the glucosylglycerol is provided in an aqueous solution.

9. Use according to any one of claims 1 to 8, **characterized in that** the concentration of glucosylglycerol ranges between 0.001 % w/w and 10 % w/w, in particular between 0.01 % w/w and 6 % w/w.

10. Glucosylglycerol for the protection of human skin and/or mucous membrane against reactive oxygen compounds, free radicals, chemicals, toxins, allergens, denaturing substances, substances binding to DNA, substances damaging proteins, substances deactivating proteins, or airborne particulate by increasing the expression of cell protective enzymes.

11. Glucosylglycerol according to claim 10, **characterized in that** the glucosylglycerol is 2-O-α-D-glucosylglycerol.

12. Glucosylglycerol according to claim 10 or 11, **characterized in that** the cell protective enzyme is an enzyme decomposing reactive oxygen compounds.

13. Glucosylglycerol according to claim 12, **characterized in that** the enzyme is a superoxide dismutase, a catalase or a glutathione peroxidase.

14. Glucosylglycerol according to claim 13, **characterized in that** the superoxide dismutase is SOD-1 or SOD-2.

15. Glucosylglycerol according to claim 10 or 11, **characterized in that** the cell protective enzyme is a DNA repairing enzyme, in particular a ligase, or a chaperone.

## Revendications

1. Utilisation de glucosylglycérole pour la fabrication d'un produit pour la protection de la peau humaine et/ou de la muqueuse contre des composés oxygénés réactifs, des radicaux libres, des produits chimiques, des toxines, des allergènes, des substances dénaturantes, des substances liant à l'ADN, des produits nuisibles aux protéines, des substances activant des protéines ou des poussières en suspension, par augmentation de l'expression d'enzymes de protection de cellules.

2. Utilisation selon la revendication 1, **caractérisée en ce que** le glucosylglycérole est du 2-O-α-D-glucosylglycérole.

3. Utilisation selon la revendication 1 ou 2, **caractérisée en ce que** l'enzyme de protection de cellules est une enzyme dégradant des composés oxygénés réactifs.

4. Utilisation selon la revendication 3, **caractérisée en ce que** l'enzyme est une superoxyde dismutase, une catalase ou une glutathion peroxydase.

5. Utilisation selon la revendication 4, **caractérisée en ce que** la superoxyde dismutase est une SOD-1 ou SOD-2.

6. Utilisation selon la revendication 1 ou 2, **caractérisée en ce que** l'enzyme de protection de cellules est une enzyme de réparation d'ADN, notamment une ligase.

7. Utilisation selon la revendication 1 ou 2, **caractérisée en ce que** l'enzyme de protection de cellules est un chaperon.

8. Utilisation selon l'une quelconque des revendications 1 à 7, **caractérisée en ce que** le glucosylglycérole se présente sous la forme d'une solution acqueuse.

9. Utilisation selon l'une quelconque des revendications précédentes, **caractérisée en ce que** la concentration du glucosylglycérole est de 0,001 % en poids à 10 en poids, notamment de 0,01 % en poids à 6 % en poids.

10. Glucosylglycérole pour la protection de la peau humaine et/ou de la muqueuse contre des composés oxygénés réactifs, des radicaux libres, des produits chimiques, des toxines, des allergènes, des substances dénaturantes, des substances liant à l'ADN, des produits nuisibles aux protéines, des substances activant des protéines ou des poussières en suspension, par augmentation de l'expression d'enzymes de protection de cellules.

11. Glucosylglycérole selon la revendication 10, **caractérisé en ce que** le glucosylglycérole est du 2-O-α-D-glucosylglycérole.

12. Glucosylglycérole selon la revendication 10 ou 11, **caractérisé en ce que** l'enzyme de protection de cellules est une enzyme dégradant des composés oxygénés réactifs.

13. Glucosylglycérole selon la revendication 12, **caractérisé en ce que** l'enzyme est une superoxyde dismutase, une catalase ou une glutathion peroxydase.

14. Glucosylglycérole selon la revendication 13, **caractérisée en ce que** la superoxyde dismutase est une SOD-1 ou SOD-2.

15. Glucosylglycérole selon la revendication 10 ou 11, **caractérisée en ce que** l'enzyme de protection de cellules est une enzyme de réparation d'ADN, notamment une ligase, ou un chaperon.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19540749 A1
- JP 2004331578 A
- WO 2008034158 A2